# EUROPEAN PATENT SPECIFICATION

(11) **EP 3 796 956 B1**
(45) Date of publication and mention of the grant of the patent: **10.08.2022**
(21) Application number: 19732738.0
(22) Date of filing: 23.05.2019
(51) Int. Cl.: A61M 1/34, A61M 1/36, B01D 15/36, A61M 1/16

(54) **EXTRACORPOREAL CIRCUIT FOR REMOVAL OF CO2 FROM BLOOD**
EXTRAKORPORALER KREISLAUF ZUR ENTFERNUNG VON CO2 AUS BLUT
CIRCUIT EXTRACORPOREL POUR L'ÉLIMINATION DU CO2 PRÉSENT DANS LE SANG

(30) Priority: 23.05.2018 IT 201800005641
(43) Date of publication of application: 31.03.2021
(73) Proprietor: UNIVERSITA' DEGLI STUDI DI MILANO, 20122 Milano (IT); FONDAZIONE IRCCS ''CA' GRANDA - OSPEDALE MAGGIORE POLICLINICO'', 20122 Milano (IT)
(72) Inventor: PESENTI, Antonio Maria, 20122 Milano (IT); ZANELLA, Alberto, 20122 Milano (IT)
(74) Representative: Casciano, Lidia Giulia Rita
(86) International application number: PCT/IB2019/054288
(87) International publication number: WO 2019/224775

(56) References cited:
- EP-A1- 2 689 791
- US-A1- 2012 035 523
- US-A1- 2017 224 898
- US-A1- 2017 348 472

## Description

### CROSS-REFERENCE TO RELATED APPLICATIONS

This patent application claims priority from Italian patent application no. 102018000005641 filed on 23/05/2018.

### TECHNICAL FIELD

The present invention relates to an extracorporeal circuit for removing CO₂ from blood.

The extracorporeal removal of CO₂ from blood is necessary when the pulmonary function has to be supplemented, even only partially, when said function is compromised due to various pathologies.

### BACKGROUND ART

In general, extracorporeal circuits for removing CO₂ from blood entail drainage of the patient's blood into an extracorporeal circuit to be artificially oxygenated and decarbonated by means of a gas exchanger, such as an oxygenator. More specifically, in a veno-venous extracorporeal system, part of the venous blood which returns towards the right atrium of the heart is collected in the extracorporeal circuit and pumped into the oxygenator, from which it is then reinfused into the venous system.

The most commonly used oxygenator devices are membrane oxygenators in which the blood and the oxygen (or an oxygen-rich mixture) are made to flow on opposite sides of a membrane. This type of apparatus therefore acts on the quantity of CO₂ dissolved in the blood which is actually a limited portion of the total CO₂ content. In fact, it is known that 90% of the CO₂ transported in the blood is in the form of bicarbonate ions according to the chemical balance (I) shown below.

CO₂ + H₂O ↔ H₂CO₃ ↔ H⁺ + HCO₃⁻ (I)

A solution for increasing removal of CO₂ from blood by means of membrane oxygenators is that of intervening on the chemical balance (I) inducing an increase in the percentage of gaseous CO₂. This increase in the concentration of gaseous CO₂ subject to removal by the oxygenator favours removal of the total CO₂.

One of the ways of intervening on the chemical balance (I) in favour of the gaseous CO₂ is exogenous acidification. Said acidification is achieved by adding an acid substance in the extracorporeal circuit upstream of the oxygenator.

By converting the bicarbonate ions into dissolved CO₂, the acidification increases the transmembrane pCO₂ gradient, and therefore increases the transfer of CO₂ through the oxygenator membrane. Exogenous acidification of the blood allows removal of the equivalent of 50% of the total production of CO₂ of an adult male from extracorporeal blood at a rate of 250 ml/min.

Alternatively, a technique based on ventilation of the acidified dialysate has also been developed, which has shown a slightly lower efficiency (10-15%) compared to direct ventilation of the blood, but reduces the exogenous contact surface with the blood and can therefore offer possible advantages.

In both cases, the acidification is obtained by infusion of a concentrated solution of metabolizable organic acids, for example lactic acid. However, when infused exogenously, despite their high clearance, said acids can cause a certain level of metabolic acidosis and an increase in the production of total corporeal CO₂, thus reducing the effectiveness of the extracorporeal removal of CO₂ in reducing the minute ventilation of the patient.

Therefore, as will be obvious to a person skilled in the art, said addition of acid can reduce the effectiveness of the extracorporeal technique of CO₂ removal and can entail a series of drawbacks that must be dealt with in order not to alter the patient's homeostasis.

Relevant prior art is for instance disclosed in documents US2017/224898 A1, US2012/035523 A1, EP2689791 A1 and US2017/348472 A1.

### DISCLOSURE OF INVENTION

The object of the present invention is therefore to produce an extracorporeal circuit for removing CO₂ from blood, able to increase removal of the CO₂ by intervening on the chemical balance (I) without recourse to the administration of an exogenous acid, while at the same time guaranteeing high safety levels.

Said object is achieved by the present invention relative to an extracorporeal circuit for removing CO₂ from blood, the essential characteristics of which are described in claim 1, and the preferred and/or auxiliary characteristics of which are described in the dependent claims.

In particular, an extracorporeal circuit is provided for removing CO₂ from blood comprising a withdrawal line for withdrawing blood from the patient, a filtration unit for the production of plasma water and a line for returning the blood to the patient defining a main circuit. The extracorporeal circuit further comprises a decarbonation group comprising means for removal of a fraction of said plasma water, a cationic resin charged with H⁺ ions adapted to generate acid plasma water, a CO₂ exchanger, means for infusion of the acid plasma water upstream of the CO₂ exchanger and means for the infusion of a solution of electrolytes downstream of said CO₂ exchanger.

Advantageously, the use of a cationic resin charged with H⁺ ions to treat the plasma water, for example a dialysate or an ultrafiltrate, allows replacement, in a controlled manner, of the positively charged ions present in said solution, without the addition of exogenous acids.

In particular, the H⁺ charged cationic resin is able to replace the calcium, sodium, potassium and magnesium ions present in the plasma water with hydrogen ions, leading to a regional acidification of this solution forming an acid solution.

The regional acidification allows improvement in the extracorporeal removal of carbon dioxide, acting on the chemical balance (I) shown above.

Furthermore, the H⁺ charged cationic resin, by removing sodium ions above all, since they are present in greater quantities in the blood, allows greater acidification of the plasma water than the systems known in the art with the same volume of liquid treated. A further advantage of the use of an H⁺ charged cationic resin consists in the fact that said resin works without the addition of exogenous liquids, simplifying the hydroelectrolytic balance of the blood after the treatment before reinfusion in the patient.

The device of the invention, acting on the concentration of cations in the blood, can furthermore be used not only for removing CO₂ but also to simultaneously carry out a continuous renal replacement therapy.

Continuous renal replacement therapy (CRRT) is an extracorporeal support technique widely used in the treatment of patients affected by acute kidney failure and other pathological conditions like sepsis and multi-organ dysfunction. During CRRT the patient's blood is treated through diffusion, conduction and absorption mechanisms.

In one embodiment, the filtration unit is a filter selected from the group consisting of a haemodialysis filter, a haemofilter and a dialyser.

Preferably the cationic resin is a resin provided with sulphonic groups or carboxylic groups.

### BRIEF DESCRIPTION OF THE DRAWINGS

For a better understanding of the invention, it will now be described in detail for purely illustrative non-limiting purposes with the help of the figures of the attached drawings, in which:
- Figure 1 illustrates a first embodiment of the invention;
- Figure 2 illustrates a second embodiment of the invention;
- Figure 3 illustrates a third embodiment of the invention;
- Figure 4 illustrates a fourth embodiment of the invention;
- Figure 5 illustrates a fifth embodiment of the invention;
- Figure 6 illustrates a sixth embodiment of the invention.

### BEST MODE FOR CARRYING OUT THE INVENTION

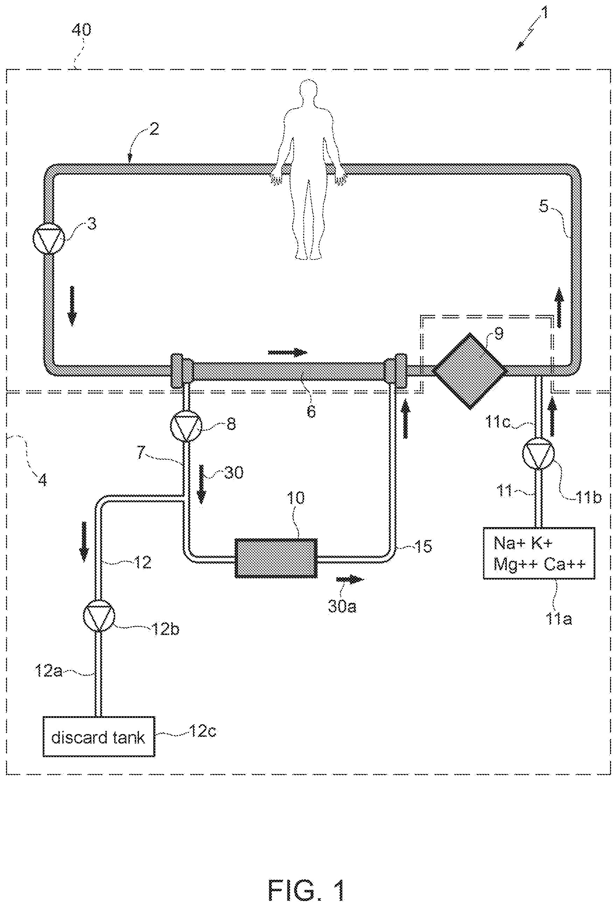

In figure 1 an extracorporeal circuit for removing blood according to a first embodiment of the present invention is indicated overall by 1.

The circuit 1 comprises a withdrawal line for withdrawing blood 2 from the patient, on which a pump 3 acts, for example a peristaltic pump, a filtration unit 6, for example a haemodialysis filter, a decarbonating group 4 and a line for returning the blood 5 to the patient. The withdrawal line for withdrawing blood 2 from the patient, the filtration unit 6, and the line for returning the blood 5 to the patient define a main circuit 40.

The haemodialysis filter 6 is connected to a secondary circuit 7 for recirculation of the plasma water 30, in this case dialysate, on which a pump 8 acts.

The decarbonating group 4 comprises a cationic resin charged with H⁺ ions 10 adapted to generate acid plasma water 30a positioned downstream of the pump 8 on the secondary circuit 7 of the plasma water 30.

The decarbonating group 4 further comprises an oxygenator 9 arranged on the main circuit 40 downstream of the haemodialysis filter 6 along the return line 5 for returning the blood to the patient.

Advantageously the cationic resin 10 is able to retain the calcium, potassium and magnesium ions but above all the sodium ions, present in large quantities in the plasma water 30, replacing them with H⁺ ions and generating acid plasma water 30a at the resin outlet.

Advantageously the acidification of the plasma water 30 causes a shift in the chemical balance (I) in favour of the gaseous CO₂ in the acid plasma water 30a in recirculation. The gaseous CO₂ is then removed by the action of the oxygenator 9, for example a bubble oxygenator. In this way, it is possible to improve removal of the CO₂ from the plasma water 30a.

The acid plasma water 30a is returned to the haemodialysis filter 6, inside which it will function as a dialysis liquid, by means of the line 15. The blood flowing out of the haemodialysis filter 6 is then deprived of the CO₂ by the oxygenator 9. The blood flowing out of the oxygenator 9 will therefore have an electrolytic imbalance due to the removal of cations by the cationic resin 10. To re-balance the concentration of the electrolytes in the blood before reinfusion to the patient, downstream of the oxygenator 9, along the line 5, means 11 are provided for the reinfusion of sodium, potassium, calcium and magnesium ions in solution to re-balance the quantity of cations lost at the level of the cationic resin 10. Said means 11 for the reinfusion of ions consist for example in one or more sacs 11a each containing an electrolytic solution, a pump 11b and an infusion line 11c. For example, an electrolytic solution comprising NaOH, KOH, NaCl or NaHCO₃ can be infused and, separately, a solution comprising calcium ions and magnesium ions. Said solutions can also include glucose if CRRT is also performed.

The circuit 1 further comprises, upstream of the cationic resin 10, means 12 for removing part of the plasma water. Said means 12 for removing part of the plasma water consist, for example, of a removal line 12a, a peristaltic pump 12b and a discard tank 12c.

The removal of part of the plasma water is necessary to compensate for the increase in volume of the plasma water, which occurs following addition of the electrolytic solution through the means 11. Therefore, to respect the hydroelectric balance of the system, the quantity of water removed by the means 12 must be equal to the quantity of water reinfused with the electrolytic solution through the means 11.

Likewise, the electrolytic solution reinfused with the means 11 must contain a quantity of sodium, potassium, calcium and magnesium equal to that removed with the cationic resin 10 plus that removed with the means 12 for removing part of the plasma water.

Alternatively, when the circuit of the invention is used, not only for removing the CO₂ but also for renal therapy, the quantity of water and electrolytes removed by the means 12 can be different from that reinfused with the means 11.

Lastly, the blood flowing out of the haemodialysis filter 6 is returned to the patient by means of a line for returning the blood 5.

In the circuit of figure 1, for example, the blood flowing out of the patient can be set to 200 ml/min, the flow of the plasma water through the cationic resin is therefore 10-25 ml/min.

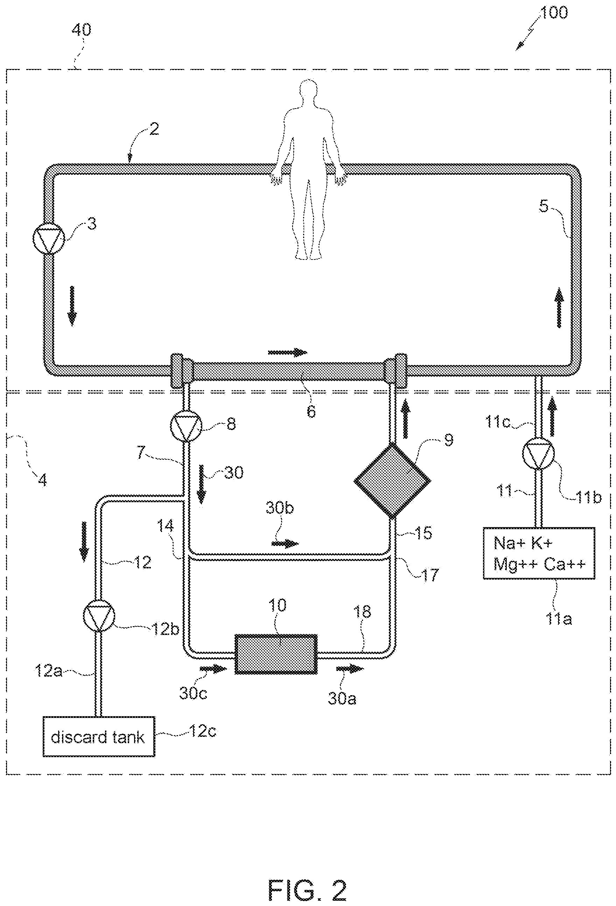

Figures 2 to 6 illustrate alternative embodiments of the circuit of figure 1 according to the present invention; the details similar or equal to those already described are indicated for the sake of simplicity by the same reference numbers.

In particular, figure 2 illustrates a second embodiment of the invention which represents a variation 100 of the circuit illustrated in figure 1. In this case, the plasma water 30 flowing out of the haemodialysis filter is split into two flows, 30b and 30c respectively, at the connection point 14. Then part of the plasma water, indicated by 30c, is treated by the cationic resin 10, while the remaining part of the plasma water 30b is sent back to the connection point 17.

The acid plasma water 30a flowing out of the cationic resin 10 is recombined by means of the line 18 with the plasma water 30b and then conveyed along the line 15 to the oxygenator 9 which, in this embodiment, is on the secondary circuit 7.

Advantageously, this embodiment reduces the exogenous contact surface between the blood and the extracorporeal circulation system and reduces the pH variations to which the blood is exposed.

In this embodiment, for example, the blood flow leaving the patient can be set to 200 ml/min and the flow of the ultrafiltrate from the haemodialysis filter can be set to 400-1000 ml/min.

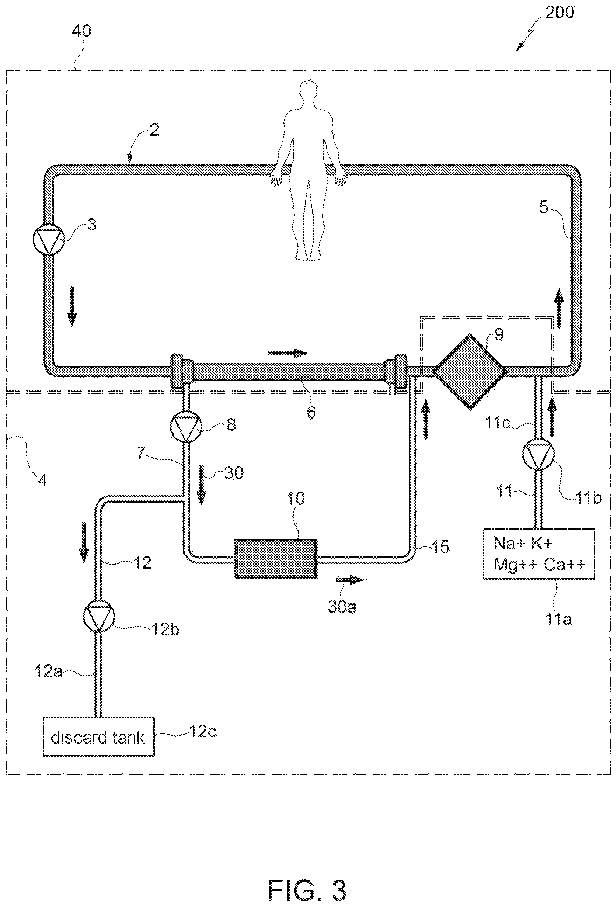

In figure 3 a third embodiment of the invention is illustrated which represents a variation 200 of the circuit illustrated in figure 1. In this case, the oxygenator 9 is positioned downstream of the haemodialysis filter 6 on the main circuit 40 on the line for return of the blood 5 to the patient and the plasma water flowing out of the cationic resin is returned to the main circuit of the blood on line 5 upstream of the oxygenator 9. In this embodiment, the oxygenator 9 is a membrane oxygenator. In this embodiment, for example, the blood flowing out of the patient can be set to 200 ml/min and the flow of the ultrafiltrate from the haemodialysis filter can be set to 10-25 ml/min.

In all the embodiments of the present invention, a gas trap can also be inserted downstream of the cationic resin 10 but upstream of the oxygenator 9 to further favour elimination of the carbon dioxide and prevent the accumulation of gas.

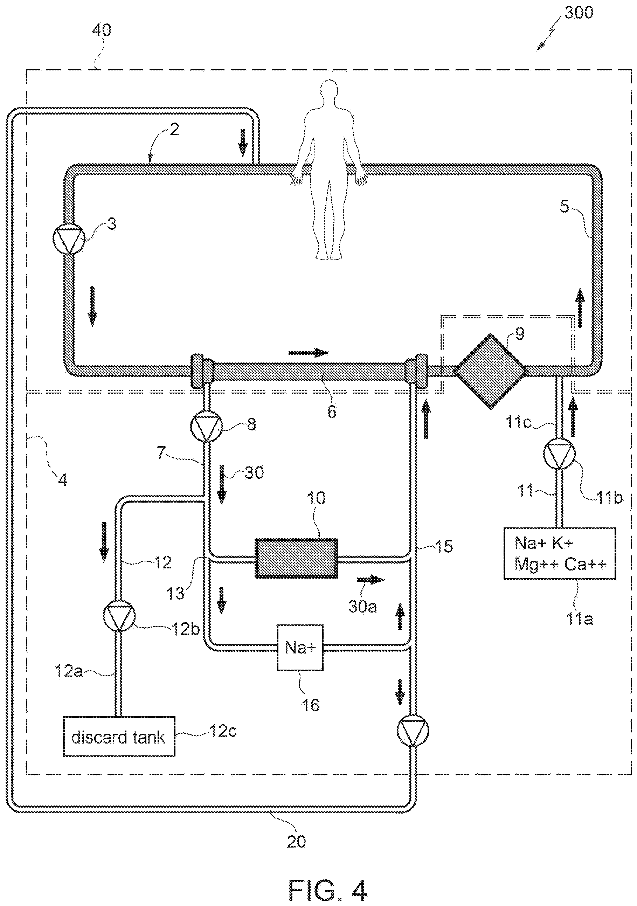

All the embodiments of the present invention can furthermore include a regional blood anticoagulation system. For example, the circuit of Figure 1 can be modified as illustrated in Figure 4. In particular, in the circuit 300, the plasma water flowing out of the haemodialysis filter 6 is split into two flows at the connection point 13. A part of the flow is directed to the cationic resin 10 while the remaining part is treated with a group for removal of the calcium, such as, for example, a cationic exchange resin 16 charged with sodium and potassium ions. Said resin 16 charged with sodium ions retains calcium ions, releasing sodium ions. The plasma water flowing out of the resin 16 charged with sodium ions is partly recombined with the acid plasma water 30a on the line 15 downstream of the cationic resin 10 and partly sent back on the line 2 upstream of the haemodialysis filter by means of the line 20 to carry out regional anticoagulation of the blood in the circuit. Restoration of the correct hydro-electrolytic balance in the blood is then carried out by the means 11 for the reinfusion of ions and the means 12 for removal of the plasma water.

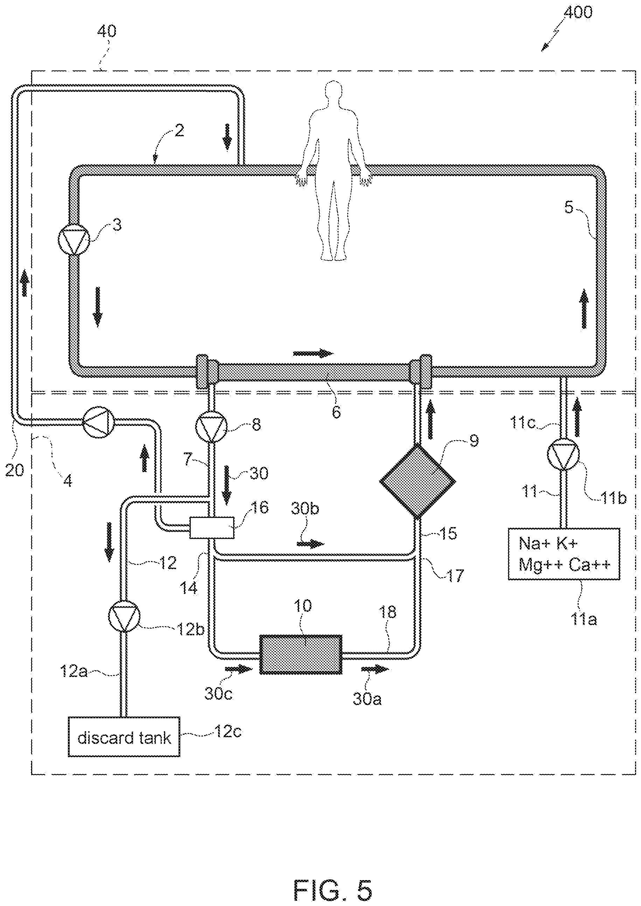

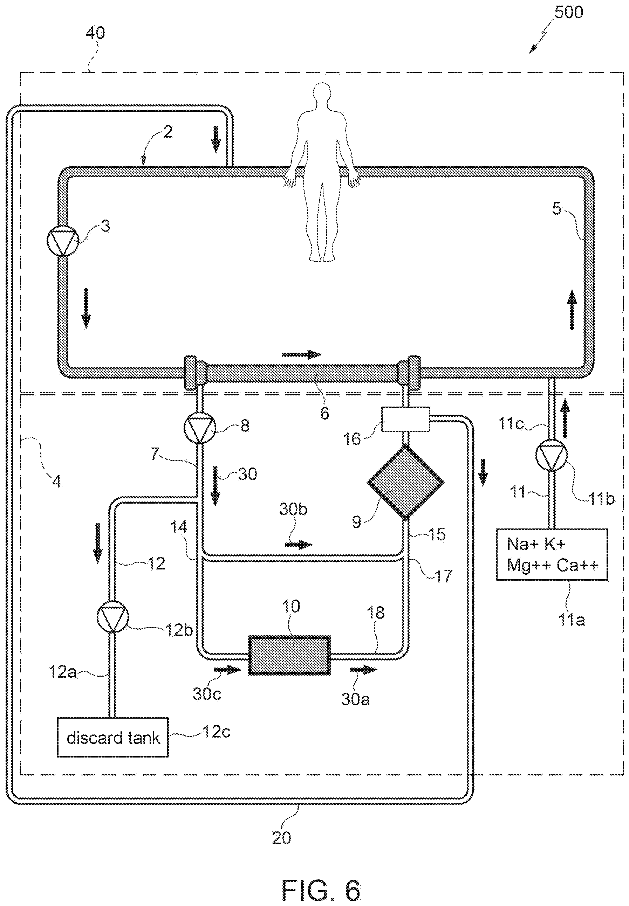

The circuit of Figure 2 can be modified as in Figures 5 and 6. In particular, in the circuit 400 of figure 5, a cationic exchange resin 16 charged with sodium and potassium ions is arranged upstream of the connection point 14 on the secondary circuit 7.

Alternatively, in the circuit 500 of figure 6, the cationic exchange resin 16 charged with sodium and potassium ions is present downstream of the oxygenator 9. In both cases part of the plasma water flowing out of the resin 16 is re-introduced into the circuit at the height of the withdrawal line for withdrawing blood from the patient 2 by means of the line 20 so as to cause regional anticoagulation of the blood.

## Claims

1. An extracorporeal circuit (1, 100, 200, 300, 400, 500) for removing CO₂ from blood comprising:
a blood withdrawal line (2) for withdrawing blood from the patient, a filtration unit (6) for producing plasma water (30) and a line for returning the blood (5) to the patient, defining a main circuit (40); and a decarbonating group (4) comprising a secondary circuit (7) for the recirculation of plasma water, means (12) for removing a fraction of said plasma water (30) and a CO₂ exchanger (9), **characterized in that** the secondary circuit (7) further comprises
a cationic resin charged with H+ ions (10) set upstream of said CO₂ exchanger (9) and adapted to generate acid plasma water (30a), means (15) for the infusion of said acid plasma water (30a) upstream of said CO₂ exchanger (9) and means (11) for the infusion of ions in a solution downstream of said CO₂ exchanger (9).

2. The extracorporeal circuit according to claim 1, **characterised in that** said filtration unit (6) is a filter selected from the group consisting of a haemodialysis filter, a haemofilter and a dialyser.

3. The extracorporeal circuit according to claim 1, **characterised in that** said cationic resin charged with H+ ions is selected from the group consisting of a cationic resin provided with sulphonic group and cationic resin provided with carboxylic groups.

4. The extracorporeal circuit according to claim 1, **characterised in that** said CO₂ exchanger (9) is present on the main circuit (40) on said line for returning the blood (5) to the patient downstream of said filtration unit (6).

5. The extracorporeal circuit according to claim 1, **characterised in that** said CO₂ exchanger (9) is present on said secondary circuit (7) downstream of said cationic resin charged with H+ ions (10).

6. The extracorporeal circuit according to any one of the preceding claims, **characterised in that** it further comprises a gas trap arranged upstream of said CO₂ exchanger (9) and downstream of said cationic resin charged with H+ ions (10).

7. The extracorporeal circuit according to any one of the preceding claims, **characterised in that** it further comprises a group for removing calcium (16) arranged on the secondary circuit (7) for the recirculation of plasma water.

8. The extracorporeal circuit according to the preceding claim, **characterised in that** said group for removing calcium is a cationic exchange resin (16) charged with sodium and potassium ions.

9. The extracorporeal circuit according to the preceding claim, **characterised in that** said group for removing calcium (16) is positioned in parallel to said cationic resin charged with H+ ions (10).

10. The extracorporeal circuit according to the preceding claim, **characterised in that** said group for removing calcium (16) is positioned in series to said cationic resin charged with H+ ions (10).

## Patentansprüche

1. Extrakorporaler Kreislauf (1, 100, 200, 300, 400, 500) zum Entfernen von CO₂ aus dem Blut, umfassend:
eine Blutentnahmeleitung (2) zur Entnahme von Blut aus dem Patienten, eine Filtrationseinheit (6) zur Herstellung von Plasmawasser (30) und eine Leitung zur Rückführung des Blutes (5) zum Patienten, wodurch ein Hauptkreislauf (40) gebildet wird; und
eine Dekarbonisierungsgruppe (4), die einen sekundären Kreislauf (7) zur Rezirkulation von Plasmawasser, Mittel (12) zur Entfernung eines Teils des Plasmawassers (30) und ein CO₂-Austauscher (9) aufweist,
**dadurch gekennzeichnet, dass** der sekundäre Kreislauf (7) ferner umfasst ein kationisches Harz, das mit H+ Ionen (10) geladen ist, stromaufwärts des C0₂-Austauschers (9) gesetzt und eingerichtet ist, saures Plasmawasser (30a) zu erzeugen, Mittel (15) zur Infusion des sauren Plasmawassers (30a) stromaufwärts des C02-Austauschers (9) und Mittel (11) zur Infusion von Ionen in eine Lösung stromabwärts des C0₂-Austauschers (9).

2. Extrakorporaler Kreislauf gemäß Anspruch 1, **dadurch gekennzeichnet, dass** die Filtrationseinheit (6) ein Filter ist, der aus der Gruppe ausgewählt ist, die aus einem Hämodialysefilter, einen Hämofilter und einen Dialysator besteht.

3. Extrakorporaler Kreislauf nach Anspruch 1, **dadurch gekennzeichnet, dass** das kationische Harz, das mit H+ Ionen geladen ist, aus der Gruppe ausgewählt ist, die aus einem kationischen Harz mit einer Sulfongruppe und einem kationischen Harz mit Carboxylgruppen besteht.

4. Extrakorporaler Kreislauf gemäß Anspruch 1, **dadurch gekennzeichnet, dass** der CO₂-Austauscher (9) im Hauptkreislauf (40) an der Leitung zur Rückführung des Blutes (5) zu dem Patienten stromabwärts der Filtrationseinheit (6) vorliegt.

5. Extrakorporaler Kreislauf gemäß Anspruch 1, **dadurch gekennzeichnet, dass** der CO₂-Austauscher (9) in dem sekundären Kreislauf (7) stromabwärts des kationischen Harzes, das mit H+-Ionen (10) geladen, vorliegt.

6. Extrakorporaler Kreislauf nach einem der vorhergehenden Ansprüche, **dadurch gekennzeichnet, dass** er weiterhin einen Gasfalle aufweist, die stromaufwärts des C0₂-Austauschers (9) und stromabwärts des kationischen Harzes, das mit H+-Ionen (10) geladenen, angeordnet ist.

7. Extrakorporaler Kreislauf nach einem der vorhergehenden Ansprüche, **dadurch gekennzeichnet, dass** er weiterhin aufweist eine Gruppe zum Entfernen von Kalzium (16), angeordnet in dem sekundären Kreislauf (7) zur Rezirkulation von Plasmawasser.

8. Extrakorporaler Kreislauf nach dem vorhergehenden Anspruch, **dadurch gekennzeichnet, dass** die Gruppe zum Entfernen von Kalzium (16) ein kationisches Austauschharz (16) ist, das mit Natrium- und Kaliumionen geladen ist.

9. Extrakorporaler Kreislauf nach dem vorhergehenden Anspruch, **dadurch gekennzeichnet, dass** die Gruppe zum Entfernen von Kalzium (16) parallel zu dem kationischen Harz (10), das mit H+-Ionen geladenen ist, angeordnet ist.

10. Extrakorporaler Kreislauf nach dem vorhergehenden Anspruch, **dadurch gekennzeichnet, dass** die Gruppe zum Entfernen von Kalzium (16) in Reihe zu dem kationischen Harz (10), das mit H+-Ionen geladenen ist, angeordnet ist.

## Revendications

1. Circuit extracorporel (1, 100, 200, 300, 400, 500) pour l'élimination du CO₂ du sang comprenant :
une conduite de prélèvement de sang (2) pour prélever du sang au patient, une unité de filtration (6) pour produire de l'eau plasmatique (30) et une conduite de retour du sang (5) vers le patient, définissant un circuit principal (40) ; et
un groupe de décarbonatation (4) comprenant un circuit secondaire (7) pour la recirculation de l'eau plasmatique, des moyens (12) pour éliminer une fraction de ladite eau plasmatique (30) et un échangeur de CO₂ (9), **caractérisé en ce que** le circuit secondaire (7) comprend en outre une résine cationique chargée d'ions H+ (10) placée en amont dudit échangeur de CO₂ (9) et adaptée pour générer de l'eau plasmatique acide (30a), des moyens (15) pour l'infusion de ladite eau plasmatique acide (30a) en amont dudit échangeur de CO₂ (9) et des moyens (11) pour l'infusion d'ions dans une solution en aval dudit échangeur de CO₂ (9).

2. Circuit extracorporel selon la revendication 1, **caractérisé en ce que** ladite unité de filtration (6) est un filtre sélectionné dans le groupe constitué d'un filtre d'hémodialyse, d'un hémofiltre et d'un dialyseur.

3. Circuit extracorporel selon la revendication 1, **caractérisé en ce que** ladite résine cationique chargée d'ions H+ est sélectionnée dans le groupe constitué d'une résine cationique pourvue d'un groupe sulfonique et d'une résine cationique pourvue de groupes carboxyliques.

4. Circuit extracorporel selon la revendication 1, **caractérisé en ce que** ledit échangeur de CO₂ (9) est présent sur le circuit principal (40) sur ladite conduite de retour du sang (5) vers le patient en aval de ladite unité de filtration (6).

5. Circuit extracorporel selon la revendication 1, **caractérisé en ce que** ledit échangeur de CO₂ (9) est présent sur ledit circuit secondaire (7) en aval de ladite résine cationique chargée d'ions H+ (10).

6. Circuit extracorporel selon l'une quelconque des revendications précédentes, **caractérisé en ce qu'**il comprend en outre un piège à gaz agencé en amont dudit échangeur de CO₂ (9) et en aval de ladite résine cationique chargée d'ions H+ (10).

7. Circuit extracorporel selon l'une quelconque des revendications précédentes, **caractérisé en ce qu'**il comprend en outre un groupe d'élimination du calcium (16) agencé sur le circuit secondaire (7) pour la recirculation de l'eau plasmatique.

8. Circuit extracorporel selon la revendication précédente, **caractérisé en ce que** ledit groupe d'élimination du calcium est une résine d'échange cationique (16) chargée d'ions sodium et d'ions potassium.

9. Circuit extracorporel selon la revendication précédente, **caractérisé en ce que** ledit groupe d'élimination calcium (16) est positionné en parallèle par rapport à ladite résine cationique chargée d'ions H+ (10).

10. Circuit extracorporel selon la revendication précédente, **caractérisé en ce que** ledit groupe d'élimination du calcium (16) est positionné en série par rapport à ladite résine cationique chargée d'ions H+ (10).
